# EUROPEAN PATENT APPLICATION

(11) **EP 4 759 929 A1**
(43) Date of publication of application: **17.06.2026**
(21) Application number: 23954626.0
(22) Date of filing: 07.10.2023
(51) Int. Cl.: C12N 15/10, C12Q 1/68, C12P 19/34, C12N 9/22

(54) **DNA POLYMERASE MUTANT AND USE THEREOF IN SEQUENCING**

(71) Applicant: MGI Tech Co., Ltd., Shenzhen, Guangdong 518083 (CN); Wuhan MGI Tech Co., Ltd., Wuhan, Hubei 430075 (CN)
(72) Inventor: HUANG, Siqian, Shenzhen, Guangdong 518083 (CN); LIU, Fen, Shenzhen, Guangdong 518083 (CN); GAO, Jing, Shenzhen, Guangdong 518083 (CN); YANG, Liyuan, Shenzhen, Guangdong 518083 (CN); BAI, Jiakun, Shenzhen, Guangdong 518083 (CN); XIAO, Yang, Shenzhen, Guangdong 518083 (CN); YANG, Juan, Shenzhen, Guangdong 518083 (CN); LIU, Yuepeng, Shenzhen, Guangdong 518083 (CN); SONG, Chengwei, Shenzhen, Guangdong 518083 (CN); CEN, Kaiyue, Shenzhen, Guangdong 518083 (CN); XU, Chongjun, Shenzhen, Guangdong 518083 (CN)
(74) Representative: Dehns
(86) International application number: PCT/CN2023/123270
(87) International publication number: WO 2025/073113

(57) **Abstract**

A DNA polymerase mutant and use thereof in sequencing. The DNA polymerase mutant includes at least three amino acid mutations at the following four sites or functionally equivalent sites, compared with a *Pyrococcus* sp. GB-D DNA polymerase exo-mutant: site 409, site 410, site 411, and site 486. The *Pyrococcus* sp. GB-D DNA polymerase exo-mutant has the amino acid sequence as set forth in SEQ ID NO: 1.

## Description

### FIELD

The present disclosure relates to the field of gene sequencing, and more particularly, to a DNA polymerase mutant and use thereof in sequencing.

### BACKGROUND

The interpretation of nucleic acid sequences is crucial for understanding functions and regulatory mechanisms of genes, as well as for many fundamental techniques applied in molecular biology. The DNA sequencing methods play a key role in this regard, and next-generation high-throughput DNA sequencing technology based on sequencing by synthesis (SBS) is currently one of the most widely used. Compared with the first-generation sequencing method, next-generation sequencing technology offers several significant advantages.

First, next-generation sequencing technology has advantages of high sequencing speed and high throughput, enabling large-scale genome sequencing in a short period of time. Second, the relatively low sequencing costs significantly reduce the price per base, making large-scale sequencing projects more economically feasible. In addition, next-generation sequencing technology exhibits superior sequencing accuracy and is capable of detecting low-frequency variants and heterozygous sites, which is of great significance for biologic research and clinical diagnosis. More importantly, the high flexibility of this technology makes it applicable to different target regions and sample types, expanding its application fields. These application fields include transcriptome sequencing, epigenetic sequencing, genome-wide association study, drug resistance detection, genetic identification, forensic analysis, genetic counseling, and medical diagnostics.

Sequencing polymerases play a crucial role in the next-generation sequencing process. The core principle of next-generation sequencing is sequencing by synthesis. That is, the DNA sequence is determined by capturing special labels (usually fluorescent molecule labels) carried by newly added bases during DNA replication. Sequencing polymerases are responsible for pairing and incorporating labeled bases with the template strand to form complementary DNA strands. This step is essential for the success of sequencing. However, natural polymerases usually exhibit low polymerization capacity towards artificially modified nucleotides or nucleotide analogs, which limits the efficiency and accuracy of sequencing.

Therefore, the DNA polymerases used for sequencing still need to be improved.

### SUMMARY

The present disclosure aims, at least in part, to solve at least one of the technical problems existing in the related art.

In view of the problems existing in current sequencing polymerases with respect to polymerization speed and sequencing quality (e.g., lag (delay) or strand bias), the inventors performed enzyme engineering modifications on the relevant active sites of thermostable family B polymerases from hyperthermophilic archaea, thereby obtaining novel DNA polymerase mutants. By improving the polymerase's efficiency in incorporating specific non-natural dNTPs, the sequencing speed and sequencing quality of the sequencing-by-synthesis (SBS) method are further enhanced.

To this end, in a first aspect, the present disclosure provides a DNA polymerase mutant. According to an embodiment of the present disclosure, the mutant includes at least three amino acid mutations at the following four sites or functionally equivalent sites, compared with a *Pyrococcus* sp. GB-D DNA polymerase exo-mutant: site 409, site 410, site 411, and site 486. The *Pyrococcus* sp. GB-D DNA polymerase exo-mutant has the amino acid sequence as set forth in SEQ ID NO: 1. This DNA polymerase mutant can effectively improve the polymerase's efficiency in incorporating specific non-natural dNTPs, thereby enhancing the sequencing speed and sequencing quality of the sequencing-by-synthesis (SBS) method.

In some embodiments, "incorporating" refers to linking a modified nucleotide to the free 5' hydroxyl group of a second nucleotide by forming a phosphodiester bond with the 3' phosphate group of the modified nucleotide. The second nucleotide to which the modified nucleotide is linked usually located at the 3' end of the polynucleotide chain. The terms "modified nucleotide" or "nucleotide analog" are synonymous and refer to a nucleotide modified at the 3' sugar hydroxyl group, in such a manner that the substituent has a size larger than that of the naturally occurring 3' hydroxyl group.

In some embodiments, the inventors have discovered that having mutations at one or two of site 409, site 410, site 411, and site 486 has a relatively small impact on enzymatic activity (Table 1).

MILDADYITEDGKPIIRIFKKENGEFKVEYDRNFRPYIYALLKDDSQIDEVR
KITAERHGKIVRIIDAEKVRKKFLGRPIEVWRLYFEHPQDVPAIRDKIREHSAVIDIFEY
DIPFAKRYLIDKGLIPMEGDEELKLLAFAIATLYHEGEEFAKGPIIMISYADEEEAKVITW
KKIDLPYVEVVSSEREMIKRFLKVIREKDPDVIITYNGDSFDLPYLVKRAEKLGIKLPL
GRDGSEPKMQRLGDMTAVEIKGRIHFDLYHVIRRTINLPTYTLEAVYEAIFGKPKEKVY
AHEIAEAWETGKGLERVAKYSMEDAKVTYELGREFFPMEAQLSRLVGQPLWDVSRSS
TGNLVEWYLLRKAYERNELAPNKPDEREYERRLRESYAGGYVKEPEKGLWEGLVSL
DFRSLYPSIIITHNVSPDTLNREGCREYDVAPEVGHKFCKDFPGFIPSLLKRLLDERQEI
KRKMKASKDPIEKKMLDYRQRAIKILANSYYGYYGYAKARWYCKECAESVTAWGR
EYIEFVRKELEEKFGFKVLYIDTDGLYATIPGAKPEEIKKKALEFVDYINAKLPGLLELE
YEGFYVRGFFVTKKKYALIDEEGKIITRGLEIVRRDWSEIAKETQAKVLEAILKHGNV
EEAVKIVKEVTEKLSKYEIPPEKLVIYEQITRPLHEYKAIGPHVAVAKRLAARGVKVRP
GMVIGYIVLRGDGPISKRAILAEEFDLRKHKYDAEYYIENQVLPAVLRILEAFGYRKED
LRWQKTKQTGLTAWLNIKKK (SEQ ID NO: 1).

It should be noted that, the amino acid positions in the amino acid sequence of the DNA polymerase mutant described in the present disclosure are determined with reference to the amino acid positions in the amino acid sequence of the wild-type *Pyrococcus* sp. GB-D DNA polymerase or wild-type *Pyrococcus* sp. GB-D DNA polymerase exo-mutant. The amino acid sequence set forth in SEQ ID NO: 1 is obtained by mutating amino acid D at site 141 to A and amino acid E at site 143 to A in the wild-type *Pyrococcus* sp. GB-D DNA polymerase. The *Pyrococcus* sp. GB-D DNA polymerase exo-mutant obtained after the mutation does not possess exonuclease activity, while the wild-type *Pyrococcus* sp. GB-D DNA polymerase possesses exonuclease activity.

It should be noted that, the term "functionally equivalent sites" include sites where, under specific circumstances, an amino acid or nucleotide mutation occurs, but the function or property of the DNA polymerase remains unchanged.

According to an embodiment of the present disclosure, the above-described DNA enzyme mutant may further include at least one of the following technical features:

According to an embodiment of the present disclosure, the DNA polymerase mutant has at least 90% identity, at least 91% identity, at least 92% identity, at least 93% identity, at least 94% identity, at least 95% identity, at least 96% identity, at least 97% identity, at least 98% identity, or at least 99% identity, or in some preferred embodiments, at least 100% identity, to the *Pyrococcus* sp. GB-D DNA polymerase exo-mutant. The DNA polymerase mutant does not include the following mutations simultaneously: L at site 409 mutated to A, Y at site 410 mutated to A, P at site 411 mutated to I, and A at site 486 mutated to L.

According to an embodiment of the present disclosure, the mutant has the following mutations:
(1) amino acid L at site 409 mutated to A, V, Y, I, F, Q, or M;
(2) amino acid Y at site 410 mutated to A or G;
(3) amino acid P at site 411 not mutated or mutated to Q, M, N, or C; and
(4) amino acid A at site 486 mutated to N, P, T, E, L, F, K, H, R, Y, M, W, or I.

According to some specific embodiments of the present disclosure, sequencing recombinases (obtained from the DNA polymerase mutants described in the present disclosure) produced by different mutation sites exhibit different relative activities, which can provide more options for actual production needs.

According to an embodiment of the present disclosure, the mutant has the following mutations: (1) amino acid L at site 409 mutated to A, V, Y, I, F, Q, or M; (2) amino acid Y at site 410 mutated to A or G; (3) amino acid P at site 411 not mutated or mutated to Q, M, N, or C; and (4) amino acid A at site 486 mutated to L, F, K, H, R, Y, M, W, or I. In some embodiments of the present disclosure, mutant combinations with enzymatic activity greater than that of the control group are preferred.

According to an embodiment of the present disclosure, the mutant has any one of mutation combinations (1) to (8):
(1) amino acid Y at site 410 mutated to G, amino acid L at site 409 mutated to V, amino acid P at site 411 not mutated, and amino acid A at site 486 mutated to L; or
(2) amino acid Y at site 410 mutated to G, amino acid L at site 409 mutated to A, amino acid P at site 411 not mutated, and amino acid A at site 486 mutated to F; or
(3) amino acid Y at site 410 mutated to G, amino acid L at site 409 mutated to V, amino acid P at site 411 not mutated, and amino acid A at site 486 mutated to F; or
(4) amino acid Y at site 410 mutated to G, amino acid L at site 409 mutated to Y, amino acid P at site 411 mutated to M, and amino acid A at site 486 mutated to K; or
(5) amino acid Y at site 410 mutated to G, amino acid L at site 409 mutated to V, amino acid P at site 411 not mutated, and amino acid A at site 486 mutated to Y; or
(6) amino acid Y at site 410 mutated to A, amino acid L at site 409 mutated to A, amino acid P at site 411 mutated to Q, and amino acid A at site 486 mutated to K; or
(7) amino acid Y at site 410 mutated to A, amino acid L at site 409 mutated to A, amino acid P at site 411 not mutated, and amino acid A at site 486 mutated to L; or
(8) amino acid Y at site 410 mutated to A, amino acid L at site 409 mutated to A, amino acid P at site 411 not mutated, and amino acid A at site 486 mutated to W.

According to an embodiment of the present disclosure, the mutant has any one of mutation combinations (1) to (11):
(1) L at site 409 mutated to A, Y at site 410 mutated to G, P at site 411 not mutated, and A at site 486 mutated to K; or
(2) L at site 409 mutated to I, Y at site 410 mutated to G, P at site 411 not mutated, and A at site 486 mutated to K; or
(3) L at site 409 mutated to A, Y at site 410 mutated to A, P at site 411 not mutated, and A at site 486 mutated to H; or
(4) L at site 409 mutated to A, Y at site 410 mutated to A, P at site 411 mutated to N, and A at site 486 mutated to K; or
(5) L at site 409 mutated to A, Y at site 410 mutated to A, P at site 411 mutated to M, and A at site 486 mutated to L; or
(6) L at site 409 mutated to M, Y at site 410 mutated to G, P at site 411 not mutated, and A at site 486 mutated to K; or
(7) L at site 409 mutated to A, Y at site 410 mutated to A, P at site 411 mutated to Q, and A at site 486 mutated to F; or
(8) L at site 409 mutated to A, Y at site 410 mutated to A, P at site 411 mutated to Q, and A at site 486 mutated to L; or
(9) L at site 409 mutated to A, Y at site 410 mutated to G, P at site 411 mutated to N, and A at site 486 mutated to K; or
(10) L at site 409 mutated to A, Y at site 410 mutated to A, P at site 411 mutated to C, and A at site 486 mutated to L; or
(11) L at site 409 mutated to A, Y at site 410 mutated to A, P at site 411 mutated to N, and A at site 486 mutated to L.

According to an embodiment of the present disclosure, the mutant has any one of mutation combinations (1) to (3):
(1) L at site 409 mutated to Y, Y at site 410 mutated to G, P at site 411 mutated to Q, and A at site 486 mutated to K; or
(2) L at site 409 mutated to I, Y at site 410 mutated to G, P at site 411 not mutated, and A at site 486 mutated to F; or
(3) L at site 409 mutated to A, Y at site 410 mutated to A, P at site 411 mutated to Q, and A at site 486 mutated to R.

According to specific embodiments of the present disclosure, some mutants may exhibit base bias in sequencing applications, and adaptive selection can be made based on actual experimental needs.

According to an embodiment of the present disclosure, the mutant has any one of mutation combinations (1) to (10):
(1) L at site 409 mutated to V, Y at site 410 mutated to G, P at site 411 not mutated, and A at site 486 mutated to K; or
(2) L at site 409 mutated to A, Y at site 410 mutated to G, P at site 411 not mutated, and A at site 486 mutated to H; or
(3) L at site 409 mutated to F, Y at site 410 mutated to G, P at site 411 not mutated, and A at site 486 mutated to F; or
(4) L at site 409 mutated to V, Y at site 410 mutated to G, P at site 411 not mutated, and A at site 486 mutated to H; or
(5) L at site 409 mutated to Q, Y at site 410 mutated to G, P at site 411 mutated to M, and A at site 486 mutated to K; or
(6) L at site 409 mutated to Y, Y at site 410 mutated to G, P at site 411 not mutated, and A at site 486 mutated to Y; or
(7) L at site 409 mutated to A, Y at site 410 mutated to G, P at site 411 not mutated, and A at site 486 mutated to M; or
(8) L at site 409 mutated to A, Y at site 410 mutated to G, P at site 411 not mutated, and A at site 486 mutated to I; or
(9) L at site 409 mutated to A, Y at site 410 mutated to G, P at site 411 mutated to M, and A at site 486 mutated to K; or
(10) L at site 409 mutated to A, Y at site 410 mutated to G, P at site 411 mutated to Q, and A at site 486 mutated to K.

According to some specific embodiments of the present disclosure, the sequencing recombinases generated by the above-described mutation sites all have higher relative activities than the control group, resulting in higher speed and better quality in actual sequencing.

According to some specific embodiments of the present disclosure, when the amino acid sequence of the DNA polymerase mutant has the above-described mutations, its relative activity is stronger than that of the recombinant polymerase obtained from the existing mutation, which can effectively incorporate non-natural dNTPs containing a fluorescently labeled 3' O-reversible terminator in the DNA strand, improving the sequencing speed and sequencing quality.

It should be noted that, the amino acid sequence of the DNA polymerase mutant described in any embodiment of the present disclosure is obtained by introducing additional mutations based on the sequence of SEQ ID NO: 1.

In a second aspect, the present disclosure provides a nucleic acid molecule. According to an embodiment of the present disclosure, the nucleic acid molecule encodes the DNA polymerase mutant described in the first aspect. The DNA polymerase mutant encoded by the nucleic acid molecule can be obtained in large quantities in vivo or in vitro.

In a third aspect of the present disclosure, the present disclosure provides an expression vector. According to an embodiment of the present disclosure, the expression vector includes the nucleic acid molecule described in the second aspect of the present disclosure.

It should be noted that, the expression vector may further include a promoter that is operably linked to the nucleic acid molecule.

According to an embodiment of the present disclosure, the expression vector is a non-pathogenic viral vector. The non-pathogenic viral vector includes an adenoviral vector or a retroviral vector.

In a fourth aspect of the present disclosure, the present disclosure provides a recombinant cell. According to an embodiment of the present disclosure, the recombinant cell carries the nucleic acid molecule described in the second aspect of the present disclosure or the expression vector described in the third aspect of the present disclosure. The recombinant cell is useful in expressing or secreting the DNA polymerase mutant described in the first aspect of the present disclosure.

According to an embodiment of the present disclosure, the recombinant cell is selected from *Escherichia coli,* yeast, or mammalian cells.

In a fifth aspect of the present disclosure, the present disclosure provides a recombinant strain. According to an embodiment of the present disclosure, the recombinant strain expresses the DNA polymerase mutant in the first aspect of the present disclosure. The DNA polymerase mutant can be rapidly obtained in large quantities by culturing the recombinant strain.

In a sixth aspect of the present disclosure, the present disclosure provides a method for obtaining a DNA polymerase mutant. According to an embodiment of the present disclosure, the method includes culturing the recombinant cell described in the fourth aspect of the present disclosure or the recombinant strain described in the fifth aspect of the present disclosure under a condition suitable for protein expression to obtain the DNA polymerase mutant.

In a seventh aspect of the present disclosure, the present disclosure provides a complex. According to an embodiment of the present disclosure, the complex includes the DNA polymerase mutant described in the first aspect of the present disclosure and a small-molecule compound or a macromolecule. The mutant is conjugated with the small-molecule compound or the macromolecule via a chemical bond.

According to an embodiment of the present disclosure, the small-molecule compound or the macromolecule includes a fluorescent label, fluorescein, or an antibody.

In an eighth aspect of the present disclosure, the present disclosure provides a method for nucleic acid synthesis. According to an embodiment of the present disclosure, the method includes: subjecting a mixture of a nucleic acid template, an amplification primer, dNTPs, and the DNA polymerase mutant described in the first aspect of the present disclosure to amplification under a condition suitable for nucleic acid amplification, to obtain the nucleic acid. The nucleic acid template can be efficiently and rapidly amplified through above-described method for nucleic acid synthesis.

It should be noted that, the DNA polymerase mutant described in the present disclosure exhibits polymerization activity for all dNTPs (including both fluorescently labeled and non-fluorescently labeled dNTPs).

In a ninth aspect of the present disclosure, the present disclosure provides a method for nucleic acid sequencing. According to an embodiment of the present disclosure, the method includes: subjecting a mixture of a nucleic acid template to be sequenced, the DNA polymerase mutant described in the first aspect of the present disclosure, and modified dNTPs to amplification under a condition suitable for nucleic acid amplification and to fluorescence signal detection; and determining the nucleic acid sequence of the nucleic acid to be sequenced based on a fluorescence signal obtained from detection.

According to an embodiment of the present disclosure, the modified dNTPs are selected from non-natural dNTPs with a fluorescently labeled 3'O-reversible terminator.

Exemplarily, the method for nucleic acid sequencing includes: mixing the nucleic acid template to be sequenced with the DNA polymerase mutant and non-natural dNTPs with a fluorescently labeled 3'O-reversible terminator, the DNA polymerase mutant being responsible for matching the non-natural dNTPs with a fluorescently labeled 3'O-reversible terminator with the nucleic acid template, and obtaining the nucleic acid sequence of the nucleic acid to be sequenced based on the nucleic acid sequence derived from the obtained fluorescent labeling signals by detecting a variety of fluorescent labeling signals. The above-described polymerization reaction and fluorescent labeling reaction can be carried out in multiple cycles based on the length of the sequencing template.

In a tenth aspect of the present disclosure, the present disclosure provides a nucleic acid sequencing kit. According to an embodiment of the present disclosure, the nucleic acid sequencing kit includes the DNA polymerase mutant described in the first aspect or the complex described in the seventh aspect. The kit described in the present disclosure is used for efficient, accurate, and rapid nucleic acid sequencing.

In an eleventh aspect of the present disclosure, the present disclosure provides use of the nucleic acid sequencing kit described in the ninth aspect in sequencing. According to an embodiment of the present disclosure, the kit can be used for sequencing, including but not limited to sequencing by synthesis (SBS) or sequencing by binding (SBB).

In a twelfth aspect of the present disclosure, the present disclosure provides use of the DNA polymerase mutant described in the first aspect, the nucleic acid molecule described in the second aspect, the expression vector described in the third aspect, the recombinant cell described in the fourth aspect, the recombinant strain described in the fifth aspect, or the complex described in the seventh aspect in the manufacture of a product for catalyzing DNA amplification or nucleic acid sequencing. According to an embodiment of the present disclosure, the DNA polymerase mutant, nucleic acid molecule, expression vector, recombinant cell, recombinant strain, or complex can be used alone or in combination to prepare products related to catalyzing DNA amplification or nucleic acid sequencing.

It should be understood that, within the scope of the present disclosure, the above-described technical features of the present disclosure and the technical features specifically described below (such as in the embodiments) can be combined with each other to form new or preferred technical solutions. Due to space limitations, they are not exhaustively described herein.

### BRIEF DESCRIPTION OF THE DRAWINGS

The above and/or additional aspects and advantages of the present disclosure will become more apparent and more readily understood from the following description of embodiments taken in conjunction with the accompanying drawings.

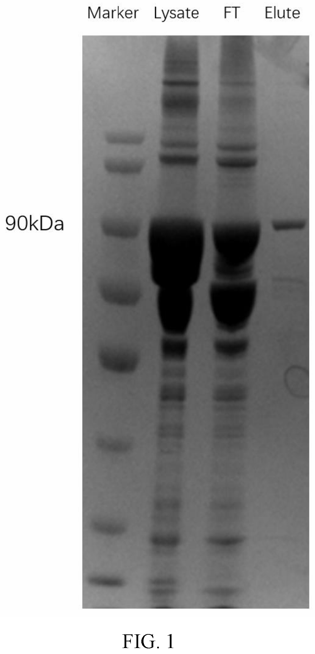

FIG. 1 shows SDS-PAGE electrophoresis results of the *Pyrococcus* sp. GB-D exo-type DNA polymerase fusion protein according to an embodiment of the present disclosure.

### DETAILED DESCRIPTION

Hereinafter, the embodiments of the present disclosure will be described in detail, embodiments of which are illustrated in the accompanying drawings. The embodiments described below with reference to the figures are exemplary and are intended to explain the present disclosure and are not to be construed as limiting the present disclosure.

In the present disclosure, unless otherwise specified, terms "first" and "second" are only used for descriptive purposes, and cannot be understood as indicating or implying relative importance or implicitly indicating the number of indicated technical features. Therefore, the features defined with "first" and "second" may explicitly or implicitly include at least one of the features. In the description of the present disclosure, "plurality" means at least two, such as two and three, unless otherwise specifically defined.

In the present disclosure, unless otherwise specified, the term "amino acid" is represented by a single-letter or three-letter code, with the following meanings: A: Ala (alanine); R: Arg (arginine); N: Asn (asparagine); D: Asp (aspartic acid); C: Cys (cysteine); Q: Gln (glutamine); E: Glu (glutamic acid); G: Gly (glycine); H: His (histidine); I: Ile (isoleucine); L: Leu (leucine); K: Lys (lysine); M: Met (methionine); F: Phe (phenylalanine); P: Pro (proline); S: Ser (serine); T: Thr (threonine); W: Trp (tryptophan); Y: Tyr (tyrosine); V: Val (valine).

In the present disclosure, unless otherwise specified, the term "nucleotide" includes both nucleotides and nucleosides. Nucleosides, like nucleotides, contain a purine or pyrimidine base linked to a ribose or deoxyribose glycoside, but lack a phosphate residue. Both synthetic and/or naturally occurring nucleotides, prior to modification of the 3' sugar hydroxyl group, are included within the definition.

In the present disclosure, unless otherwise specified, the term "homology" has the conventional meaning in the art and refers to the "identity" between two nucleic acid or amino acid sequences. Its percentage represents the statistically significant percentage of identical nucleotide or amino acid residues between the two sequences to be compared after best alignment, and differences between the two sequences are randomly distributed throughout their entire length. In the present disclosure, the mutants are described based on their mutations at specific residues, and the sites of these mutations are determined with reference to the sites of amino acids in the amino acid sequence of the wild-type *Pyrococcus* sp. GB-D DNA polymerase or the wild-type *Pyrococcus* sp. GB-D DNA polymerase exo-mutant.

In the present disclosure, unless otherwise specified, the term "relative activity" refers to the calculated enzymatic activity of each experimental group relative to the control group, when the enzymatic activity of the control group is considered to be 100%. The control group is a mutant of *Pyrococcus* sp. GB-D DNA polymerase exo-mutant (SEQ ID NO: 1) with the following amino acid mutations: L409A-Y410A-P411I-A486L, and the experimental groups are mutants of *Pyrococcus* sp. GB-D DNA polymerase mutant (SEQ ID NO: 1) with specific amino acid mutations in the embodiments of the present disclosure.

In the present disclosure, unless otherwise specified, the term "transformation" refers to the introduction of DNA into a host cell, to allow the DNA to be replicated as an extrachromosomal element or through chromosomal integration. That is, transformation refers to the heritable change in a gene caused by the introduction of exogenous DNA into the cell.

Previous studies have reported that DNA polymerases can be divided into three families, namely family A, family B, and family C. Although polymerases of families A and B share structurally similar nucleotide-binding sites, the motifs among different polymerase families are significantly distinct and thus represent different recognition mechanisms for nucleotides and their analogs. It is particularly noteworthy that the thermostable family B polymerases derived from hyperthermophilic archaea exhibit excellent incorporation performance for various nucleotide analogues. These thermostable family B polymerases include KOD (*Thermococcus kodakaraensis*), 9°N (*Thermococcus sp. 9*°*N*), TGO (*Thermococcus gorgonarius*), TOK (*Desulfurococcus sp. Tok*), Vent DNA polymerase (*Thermococcus litoralis*), JDF-3, and Pfu DNA polymerase (*Pyrococcus furiosus*), among others.

To improve the polymerization speed and sequencing quality of existing DNA polymerases in high-throughput sequencing processes (e.g., sequencing by synthesis (SBS)), the inventors have discovered through research that although the main amino acid sequences of the DNA polymerase active sites of these enzymes remain conserved across different families, their incorporation efficiency for specific non-natural dNTPs (e.g., non-natural dNTPs containing a 3'O-reversible terminator) can be enhanced through enzyme engineering. Therefore, this approach is expected to improve the enzyme's ability to recognize and polymerize non-natural specific dNTPs while maintaining the activity of DNA polymerase.

By conducting protein spatial structure analysis and simulation calculations on the sequence of the wild-type polymerase, the inventors identified regions in the *Pyrococcus* sp. GB-D DNA polymerase associated with non-natural dNTPs carrying fluorescent labels and 3'O-reversible terminators, and performed targeted rational design on these regions. The design includes: on the one hand, mutations at L409, Y410, and P411 to eliminate the steric gate effect at these sites, thereby improving the incorporation efficiency of non-natural dNTPs with 3'O-reversible terminators that have large steric hindrance (such as methylazido); on the other hand, mutations at A486 in the finger region to stabilize the ternary ajar conformation of the non-natural dNTPs with polymerase + DNA template + DNA primer, thereby reducing the activation energy required for the polymerization reaction, which allows non-natural dNTPs with fluorescent groups and reversible terminators to more easily undergo polymerization with DNA in the enzyme.

It is known that among other family B polymerases (e.g., 9°N, Pfu, Vent), the mutant combination with good 3'O-methylazido-dNTP polymerization activity is L409A-Y410A-P411I-A486L (patent US8460910B2).

In the present disclosure, the mutant combination L409A-Y410A-P411I-A486L, which exhibits good 3'O-methylazido-dNTP polymerization activity, was used as a control group for further screening and optimization. This aims to find DNA polymerase with superior performance to more effectively interact with non-natural dNTPs carrying fluorescent labels and 3'O-reversible terminators, providing better conditions and efficiency for subsequent experiments.

In view of this, in one aspect, the present disclosure provides a DNA polymerase mutant. The mutant has at least three amino acid mutations at the following four sites or functionally equivalent sites, compared with a *Pyrococcus* sp. GB-D DNA polymerase exo-mutant: site 409, site 410, site 411, and site 486. The *Pyrococcus* sp. GB-D DNA polymerase exo-mutant has the amino acid sequence as set forth in SEQ ID NO: 1. The DNA polymerase mutant does not include mutation of L at site 409 to A, mutation of Y at site 410 to A, mutation of P at site 411 to I, and mutation of A at site 486 to L. This DNA polymerase mutant possesses significant advantages in activity and thermostability, and can effectively enhance the performance of non-natural dNTPs in polymerizing 3'O-reversible terminators, thereby significantly improving the sequencing speed and sequencing quality.

In another aspect of the present disclosure, the present disclosure provides a nucleic acid molecule encoding the above-described DNA polymerase mutant.

It should be noted that, for the nucleic acid molecules mentioned in the specification and claims of the present disclosure, those skilled in the art should understand that they actually include any one or two of the complementary double strands. In this specification and claims, for convenience, only one strand is given in most cases, but the other strand complementary to the one strand is actually also disclosed. In addition, the nucleic acid sequences of the present disclosure include a DNA form or an RNA form, the disclosure of one of which implies the disclosure of the other.

In yet another aspect of the present disclosure, the present disclosure provides an expression vector including the above-described nucleic acid molecule. The type of expression vector herein is not particularly limited, as long as it can replicate and express the mutant in a host cell.

In yet another aspect of the present disclosure, the present disclosure provides a recombinant cell, carrying the above-described nucleic acid molecule or expression vector, or expressing the DNA polymerase mutant. The recombinant cell is obtained by transfecting or transforming the expression vector. According to some specific embodiments of the present disclosure, the recombinant cell can efficiently express the above-described DNA polymerase mutant under suitable conditions.

In yet another aspect of the present disclosure, the present disclosure provides a recombinant strain expressing the above-described DNA polymerase mutant. The recombinant strain can proliferate in large quantities within a short period of time, efficiently preparing the DNA polymerase mutant.

In yet another aspect of the present disclosure, the present disclosure provides a method for obtaining a DNA polymerase mutant. The method includes culturing the above-described recombinant cell under a condition suitable for protein expression to obtain the DNA polymerase mutant.

In yet still another aspect of the present disclosure, the present disclosure provides a complex. The complex includes the above-described DNA polymerase mutant and a small-molecule compound or a macromolecule. The DNA polymerase mutant is conjugated with the small-molecule compound or the macromolecule via a chemical bond. In the field of gene sequencing, the nucleic acid sequence to be sequenced is determined by detecting the small-molecule compound (e.g., fluorescent label) or macromolecule (e.g., antibody, luciferin) in the complex.

It should be noted that, the application scenarios of the DNA polymerase mutant and complex provided in the present disclosure include, but are not limited to, nucleic acid synthesis and nucleic acid sequencing. They can also be used to screen drugs targeting viruses or cell division, and have considerable development prospects in forensic medicine and criminology.

The present disclosure is described below with reference to specific examples. It should be noted that these examples are merely illustrative and do not limit the present disclosure in any way. If the specific technologies or conditions are not specified in the examples, they shall be carried out according to the technologies or conditions as described in the literature in the art or according to the product instructions. The reagents and instruments used without indicating the manufacturer are all conventional products that can be purchased commercially.

### Example 1

This example is based on the preparation and preliminary enzymatic activity screening of *Pyrococcus* sp. GB-D exo-type DNA polymerase and its mutants. The specific steps are as follows:

### 1. Construction and Transformation of Recombinant Expression Vector

To construct the recombinant expression vector, Sangon Biotech (Shanghai) Co., Ltd. synthesized the *Pyrococcus* sp. GB-D exo-type DNA polymerase gene (SEQ ID NO: 2) fused at the C-terminus with a 6×His tag, cloned into the pD441-WT vector, as well as site-directed mutagenesis primers targeting sites 409, 410, 411, and 486.

The DNA sequence of the wild-type *Pyrococcus* sp. GB-D DNA polymerase exo-mutant (WT-Exo-) was set forth in SEQ ID NO: 2:

ATGATTCTAGACGCTGATTATATAACAGAAGACGGCAAACCGATCATCCG
CATCTTCAAGAAGGAAAACGGTGAATTTAAAGTGGAATACGATCGTAACTTTCGTC
CGTATATTTATGCGCTGCTGAAAGATGATTCGCAAATTGACGAAGTGCGCAAAATTA
CGGCCGAGCGCCACGGCAAAATCGTGCGCATTATCGACGCAGAAAAAGTCCGTAA
AAAGTTTCTGGGCCGTCCGATCGAAGTTTGGCGTTTGTACTTCGAGCACCCGCAA
GATGTTCCGGCAATCCGTGATAAAATCCGCGAGCACTCTGCTGTCATCGACATCTTT
GAGTATGACATTCCGTTTGCAAAACGTTATCTGATCGACAAAGGTCTTATCCCGATG
GAAGGAGACGAAGAACTGAAGTTACTGGCGTTTGCGATCGCCACTCTGTACCATG
AAGGTGAAGAGTTCGCTAAGGGTCCGATTATTATGATTTCCTACGCGGATGAGGAA
GAAGCTAAAGTTATCACTTGGAAAAAGATCGACCTGCCGTATGTTGAGGTAGTTTC
CTCCGAAAGAGAAATGATCAAGCGTTTTCTGAAAGTCATTCGCGAGAAGGACCCG
GATGTTATTATCACCTACAATGGTGACTCGTTCGACCTGCCGTACCTGGTTAAGCGG
GCAGAAAAGTTAGGCATTAAACTCCCACTGGGCCGTGATGGTAGCGAACCGAAAA
TGCAACGTTTGGGTGATATGACCGCTGTGGAGATTAAAGGCCGCATCCACTTTGAT
CTGTATCACGTGATTCGTCGGACCATTAACCTGCCGACCTATACCTTGGAGGCGGT
CTATGAGGCGATCTTCGGCAAACCTAAAGAGAAGGTTTATGCGCATGAGATCGCTG
AAGCCTGGGAAACCGGTAAAGGCTTGGAGCGCGTGGCGAAGTACAGCATGGAAG
ATGCGAAGGTGACGTACGAGCTGGGTCGTGAATTCTTCCCGATGGAGGCTCAGCT
GTCTCGTTTGGTTGGTCAGCCGCTGTGGGATGTTTCTCGTAGCAGCACCGGAAACC
TGGTCGAGTGGTATCTGCTTCGCAAGGCGTATGAACGCAACGAGTTGGCGCCAAAT
AAACCGGATGAAAGAGAATACGAGCGTCGCTTACGTGAAAGCTATGCCGGTGGTT

ATGTTAAGGAGCCGGAGAAGGGCCTGTGGGAAGGTCTGGTCAGCCTCGACTTCCG
CAGCCTTTATCCGTCAATTATCATCACGCATAACGTGTCCCCGGATACCTTGAATCG
TGAGGGGTGCCGTGAATACGACGTGGCTCCGGAGGTGGGTCATAAATTCTGCAAG
GATTTTCCGGGCTTCATTCCGAGCCTGCTGAAGCGTCTCTTGGACGAGCGTCAAGA
AATCAAGCGGAAAATGAAGGCGAGCAAAGACCCGATTGAAAAGAAGATGCTGGA
CTACCGCCAAAGAGCGATTAAGATCCTGGCTAATAGCTACTATGGTTATTACGGCTA
CGCGAAAGCGCGTTGGTATTGTAAAGAGTGCGCAGAGAGCGTTACTGCGTGGGGT
CGTGAGTACATTGAATTCGTTCGTAAAGAATTAGAGGAAAAGTTTGGTTTTAAGGT
CCTGTACATTGACACCGATGGGCTTTACGCCACCATTCCGGGCGCAAAGCCGGAG
GAGATCAAAAAGAAGGCGCTGGAGTTCGTGGATTATATCAACGCAAAGCTCCCGG
GCTTGCTTGAGCTGGAGTACGAGGGTTTCTATGTGCGTGGTTTTTTCGTGACTAAA
AAAAAGTATGCGCTGATAGACGAGGAGGGCAAAATCATTACGCGTGGCTTGGAGA
TCGTGCGCCGTGACTGGTCTGAGATCGCGAAGGAGACCCAGGCAAAGGTGCTGG
AAGCCATCCTGAAACACGGTAATGTGGAAGAAGCCGTTAAGATTGTCAAAGAGGT
GACCGAAAAGCTGAGCAAATACGAGATCCCACCGGAAAAGCTTGTTATTTATGAG
CAGATTACGCGTCCACTGCACGAATACAAAGCTATCGGTCCGCATGTTGCGGTGGC
CAAACGCTTGGCTGCGCGTGGTGTTAAGGTTCGTCCGGGCATGGTTATCGGCTACA
TCGTTTTGCGCGGTGACGGCCCTATTTCCAAACGTGCAATCCTGGCGGAAGAGTTC
GACCTGAGGAAGCACAAATACGACGCCGAATACTACATCGAGAACCAGGTGCTGC
CGGCAGTACTGCGTATTCTAGAGGCGTTTGGCTATCGTAAGGAGGATCTGCGTTGG
CAGAAAACCAAGCAGACCGGTCTGACCGCGTGGCTGAACATTAAAAAGAAA (SEQ
ID NO: 2).

The primers were designed as follows: 5'-reverse complementary region (15-21 bp)-non-complementary region (at least 15 bp). The target plasmid was amplified using Phanta Max Super-Fidelity DNA Polymerase.

The amino acids at site 409, site 410, site 411, and site 486 were mutated as follows:
the amino acid at site 409 was mutated to any amino acid;
the amino acid at site 410 was mutated to A or G;
the amino acid at site 411 was either wild-type P, or mutated to one of C, E, M, N, or Q; and
the amino acid at site 486 was mutated to any amino acid.

Among currently known family B DNA polymerases (such as 9°N, Pfu, Vent, etc.), a mutant combination exhibiting high activity for polymerizing non-natural dNTPs with a 3'O-reversible terminator includes L409A-Y410A-P411I-A486L (see EP1664287B1 and US8460910B2). In this example, this mutant combination was named Mut-2-1 (control group) and used as the control mutant site combination in the screening experiment of *Pyrococcus* sp. GB-D exo-type DNA polymerases.

Since the amplification product contained the original template plasmid, in order to avoid false-positive transformants after transformation, Dpn I digestion must be performed prior to recombination cyclization to remove the methylated template plasmid.

The recombinant polymerase mutant expression vector was introduced into *E. coli* BL21 competent cells and plated on selective plates (containing kanamycin 50 µg/ml) to screen for positive colonies. After overnight culture, single colonies from the experimental groups were inoculated into 5 ml LB culture medium (containing kanamycin 50 µg/ml) and cultured at 37°C, 200 rpm/min overnight. On the following day, the culture was diluted 1:100 and inoculated into 96-well plates containing 150 µl fresh LB medium (containing kanamycin 50 µg/ml), followed by incubation at 37°C with shaking at 220 rpm until OD600 = 0.6.

### 2. Induced Expression of Recombinant Protein and Bacterial Lysis

In the above-described well plate, IPTG was added to a final concentration of 0.5 mM and cultured at 25°C and 220 rpm/min for 12 h to 16 h to induce expression. Then, 1 µL of 100 mM PMSF (a protease inhibitor) and 5 µL of 10 mg/ml lysozyme were added. The mixture was gently mixed well with a pipette tip, and then incubated at 37°C for 10 min to obtain the crude bacterial lysate containing the enzyme.

### 3. FRET assay for recombinant protein and incorporation efficiency for non-natural dNTPs

In the above-described crude bacterial lysate containing the enzyme, AF532 fluorescent dye-labeled 3'O-azidomethyl-blocked modified dATP (MGI Tech Co., Ltd, patent US10988501B2) and Cy5 fluorescent dye-labeled DNA strand (Sangon Biotech (Shanghai) Co., Ltd.). The sequence information is as follows:
F: Cy5-CGTGTATGCGTAATAGGATCCCGACTCACTATGGACG (SEQ ID NO: 3);
R: Cy5-CGTGTATCGTCCATAGTGAGTCGGGATCCTATTACGC (SEQ ID NO: 4);

The incorporation of modified nucleotides during high-throughput sequencing was simulated. The relative reaction rates of polymerase mutants were determined using a microplate reader, and the crude enzymatic activity was calculated. The specific experimental methods were as follows: Reaction Buffer: 20 mM Tris-HCl, 10 mM (NH₄)₂SO₄, 10 mM KCl, 2 mM MgSO₄, pH 8.5@25°C.

Reaction system: 1 µg of different polymerase proteins obtained in step 2 were each mixed with a reaction solution containing 2 µM modified dATP and 1 µM DNA-Cy5.

Reaction solution composition: 20 mM Tris-HCl, 10 mM (NH₄)₂SO₄, 10 mM KCl, 2 mM MgSO₄, 2 µM 3'-blocked modified dATP, 1 µM DNA-Cy5, and balance water, pH 8.5.
Reaction temperature: 60°C
Reaction time: 1 h.

After the reaction was completed, data tables and enzymatic activity curves can be directly exported, and the relative activity (Relative Vmax/Km) of the crude enzyme was calculated by comparing with the enzyme mutant of the control group (Mut-2-1). The results, as illustrated in Table 1, showed that the wild-type *Pyrococcus* sp. GB-D DNA polymerase (WT-Exo-) failed to incorporate the AF532 fluorescent dye-labeled 3'O-azidomethyl-blocked modified dATP, whereas some mutant proteins were capable of incorporation and generated fluorescent signal values. It can be seen from the experimental results that different mutants exhibited varying polymerization activities towards the modified 3'-blocked dNTPs, indicating effective mutations and site combinations at different sites. Mutation site combinations with a Ralative Vmax/Km value higher than that of the control group were identified as mutants capable of efficiently incorporating 3'-blocked dNTPs. These mutants provide high potential for high-throughput sequencing. Some mutation combinations at four sites in the DNA polymerase mutants are shown in Table 1, and the control mutant Mut-2-1 combination is L409A-Y410A-P411I-A486L.

**Table 1: Combination Types of DNA Polymerase Mutants**

| **Mutant** | **L409** | **Y410** | **P411** | **A486** | **Ralative Vmax/Km** |
|---|---|---|---|---|---|
| WT-Exo-(wild type) | L | Y | P | A | 0.00 |
| Mut-2-1 (control group) | A | A | I | L | 1.00 |
| Mut-2-806 | V | G | P | L | 1.66 |
| Mut-2-829 | A | G | P | F | 1.65 |
| Mut-2-820 | V | G | P | F | 1.56 |
| Mut-2-1026 | Y | G | Q | K | 1.50 |
| Mut-2-834 | A | G | P | K | 1.45 |
| Mut-2-816 | I | G | P | F | 1.41 |
| Mut-2-875 | I | G | P | K | 1.40 |
| Mut-2-1024 | Y | G | M | K | 1.37 |
| Mut-2-937 | A | A | P | H | 1.34 |
| Mut-2-953 | A | A | N | K | 1.29 |
| Mut-2-927 | A | A | Q | R | 1.26 |
| Mut-2-957 | A | A | Q | K | 1.25 |
| Mut-2-69 | A | A | M | L | 1.22 |
| Mut-2-851 | V | G | P | Y | 1.22 |
| Mut-2-879 | V | G | P | K | 1.21 |
| Mut-2-876 | M | G | P | K | 1.20 |
| Mut-2-955 | A | A | Q | F | 1.18 |
| Mut-2-833 | A | G | P | H | 1.17 |
| Mut-2-71 | A | A | Q | L | 1.17 |
| Mut-2-815 | F | G | P | F | 1.16 |
| Mut-2-897 | V | G | P | H | 1.15 |
| Mut-2-62 | A | A | P | L | 1.11 |
| Mut-2-1015 | A | G | N | K | 1.09 |
| Mut-2-68 | A | A | C | L | 1.09 |
| Mut-2-70 | A | A | N | L | 1.09 |
| Mut-2-1021 | Q | G | M | K | 1.08 |
| Mut-2-852 | Y | G | P | Y | 1.08 |
| Mut-2-835 | A | G | P | M | 1.06 |
| Mut-2-643 | A | A | P | W | 1.05 |
| Mut-2-830 | A | G | P | I | 1.05 |
| Mut-2-1014 | A | G | M | K | 1.03 |
| Mut-2-1016 | A | G | Q | K | 1.03 |
| Mut-2-880 | Y | G | P | K | 0.98 |
| Mut-2-943 | A | A | P | T | 0.87 |
| Mut-2-808 | F | G | P | I | 0.84 |
| Mut-2-910 | A | A | M | E | 0.83 |
| Mut-2-895 | Q | G | P | H | 0.80 |
| Mut-2-941 | A | A | P | N | 0.78 |
| Mut-2-911 | A | A | M | F | 0.67 |
| Mut-2-929 | A | A | Q | M | 0.61 |
| Mut-2-644 | A | A | P | T | 0.59 |
| Mut-2-939 | A | A | P | K | 0.57 |
| Mut-2-651 | A | A | P | L | 0.55 |
| Mut-2-1047 | A | G | M | Y | 0.53 |
| Mut-2-647 | A | A | P | P | 0.50 |
| Mut-2-936 | A | A | P | F | 0.40 |
| Mut-2-855 | I | G | P | T | 0.37 |
| Mut-2-20 | L | Y | P | L | 0.20 |
| Mut-2-648 | A | A | P | H | 0.19 |
| Mut-2-26 | A | Y | P | L | 0.00 |

### Example 2

In this example, the expression and purification of the DNA polymerase mutant fusion proteins were carried out based on the *Pyrococcus* sp. GB-D exo-type DNA polymerase mutant. The specific steps are as follows:
1) Single colonies of BL21/pD441 carrying the mutant were picked, inoculated into 50 ml of LB liquid medium (containing 50 µg/ml kanamycin), and cultured overnight at 37°C with shaking at 220 rpm/min. On the next day, the culture was diluted at a ratio of 1:100 and transferred to 1000 ml of LB liquid medium (containing 50 µg/ml kanamycin). The culture was cultured at 37°C with shaking at 220 rpm/min until the OD600 reached 0.5 to 0.8. IPTG was added to a final concentration of 0.5 mM, and the culture was induced overnight at 25°C. The induced BL21/pD441 bacterial culture was collected. In addition, a blank control group without IPTG addition was set up, and the non-induced BL21/pD441 bacterial culture was collected.
2) The induced BL21/pD441 bacterial culture from step 1) was centrifuged at 8000 rpm/min for 10 min. The supernatant was discarded, and the bacterial pellet was collected and resuspended in buffer 1 (50 mM KPO₄, 500 mM NaCl, 10 mM imidazole, 5% glycerol, pH 7.0) supplemented with PMSF (a final concentration of 0.5 mM), Triton X-100 (a final concentration of 0.5%), and lysozyme (a final concentration of 0.25%). Then, the mixture was incubated at room temperature for 30 min, centrifuged at 12000 rpm/min and 4°C for 30 min, and subjected to an ice bath and ultrasonication, followed by centrifugation at 12000 rpm/min for 30 min. The supernatant obtained from centrifugation was placed in a 75°C water bath for 20 min with periodic mixing to ensure uniform heating, and centrifuged at 12000 rpm/min at 4°C for 30 min. The supernatant was filtered through a 0.22 µm filter membrane to obtain the crude extract of the fusion protein.
3) The crude extract of the fusion protein obtained in step 2) was loaded onto a Ni-affinity chromatography column (affinity chromatography prepacked column: HisTrap FF, 5 ml, 17-5255-01, GE Healthcare) at an appropriate flow rate. After loading, the column was equilibrated with 5 CV (column volumes) of buffer 1 and then eluted with 5 CV of 3% buffer 2 (50 mM KPO₄, 1 M NaCl, 5% glycerol, pH 7.0) followed by 5 CV of 50% buffer 2. The Ni-affinity chromatography eluate corresponding to the peak values greater than or equal to 100 mAU was collected.
4) The eluate corresponding to the peak value greater than or equal to 100 mAU was loaded at a certain flow rate for ion exchange chromatography (ion exchange prepacked column: HiTrap Q HP, 5 ml, 17-1154-01, GE Healthcare). After loading, the column was equilibrated with 5 CV of buffer 2, followed by linear elution with a gradient from 0% buffer 2 to 60% buffer 2. The ion exchange chromatography eluate corresponding to the peak value greater than or equal to 100 mAU was collected.
5) The ion exchange chromatography eluent corresponding to the peak value greater than or equal to 100 mAU was subjected to gel chromatography (gel chromatography prepacked column: HiPrep Sephacryl S-100HR, 26 mm, 17-1194-01, GE Healthcare). The column was first washed with 3 CV of 20% ethanol followed by 3 CV of water, and then equilibrated with 3 CV of 100% buffer 3 (20 mM Tris, 200 mM KCl, 0.2 mM EDTA, 10% glycerol, pH 7.4). The samples were loaded and eluted with 1.5 CV of buffer 3. The eluate collected corresponded to the purified wild-type DNA polymerase fusion protein.
6) The *Pyrococcus* sp. GB-D exo-type DNA polymerase fusion protein was subjected to SDS-PAGE (5% stacking gel and 12% separating gel). The results, as illustrated in FIG. 1, showed that lane 1 was the protein marker (PageRuler Prestained Protein Ladder, 26616, Thermo Scientific), lane 2 was 5 µl of the induced lysate from BL21/pD441 bacterial culture carrying the *Pyrococcus* sp. GB-D exo-type DNA polymerase mutant fusion plasmid mixed with 10 µl of 2× loading buffer, lane 3 was 5 µl of flow-through from the Ni-affinity chromatography of the lysate mixed with 10 µl of 2x loading buffer after, and lane 4 was 0.5 µl of the *Pyrococcus* sp. GB-D exo-type DNA polymerase mutant fusion protein purified by Ni-affinity chromatography mixed with 1 µl of 2x loading buffer. It can be seen that the proteins in lane 2, lane 3, and lane 4 was approximately 90 kDa, which was consistent with the molecular weight reported in the literature (Hikida Y, Kimoto M, Hirao I, Yokoyama S. Crystal structure of Deep Vent DNA polymerase. Biochemical and biophysical research communications. 2017 Jan 29;483(1):52-7.). Protein purity was analyzed using Quantity One software on the electrophoresis gel, showing that the purity of the purified DNA polymerase mutant fusion protein reached 90% or higher. The target protein of approximately 90 kDa was not observed in the non-induced BL21/pD441 bacterial culture.

### Example 3

In this example, the actual sequencing performance of the purified enzyme of the *Pyrococcus* sp. GB-D exo-type DNA polymerase mutant was evaluated on a sequencer. Specifically, the purified enzyme of the dominant mutant was subjected to sequencing experiments on the MGI SEQ-2000 sequencer, and the sequencing results were analyzed. The specific experimental steps are as follows:
Sequencing library: *E. coli.fa* library (Part No. 1000005038, MGI Tech co., Ltd);
Sequencing conditions: Polymerization time of 60 seconds, cleavage time of 60 seconds.

Using the same sequencing library *(E. coli.fa* library) and sequencing conditions, the control Mut1 mutant and five mutants that exhibited varying performance in enzymatic activity test were selected for on-machine sequencing. These mutants underwent 100 sequencing cycles (SE100) on the MGISEQ-2000 sequencer. The test results, as illustrated in Table 2, indicate that the purified enzyme of the dominant mutant exhibited superior performance in actual sequencing.

**Table 2**

| **Mutants** | **Mut-2-1** | **Mut-2-6 2** | **Mut-2-6 8** | **Mut-2-7 0** | **Mut-2-64 3** | **Mut-2-80 6** | **Mut-2-82 0** | **Mut-2-94 3** |
|---|---|---|---|---|---|---|---|---|
| Q30(%) | 89.92 | 94.74 | 93.62 | 93.65 | 94.69 | 96.13 | 97.01 | 85.55 |
| Avg Error Rate, % | 0.43 | 0.19 | 0.3 | 0.26 | 0.2 | 0.14 | 0.13 | 0.64 |
| Lag, % | 0.16 | 0.16 | 0.06 | 0.16 | 0.1 | 0.1 | 0.06 | 0.21 |
| Runon, % | 0.09 | 0.05 | 0.05 | 0.06 | 0.08 | 0.06 | 0.04 | 0.15 |
| Total Reads, M | 542.14 | 518.92 | 521.69 | 549.37 | 506.88 | 557.56 | 521.02 | 528.26 |
| Q30 decrease | 12.6 | 2.9 | 4.77 | 8.8 | 2.95 | 3.96 | 2.35 | 21.33 |

Reference throughout this specification to terms such as "an embodiment", "some embodiments", "an example", "a specific example", or "some examples" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples. In addition, different embodiments or examples and features of different embodiments or examples described in the specification may be combined by those skilled in the art without mutual contradiction.

Although embodiments of the present disclosure have been shown and described above, it should be understood that above embodiments are merely exemplary, and cannot be construed to limit the present disclosure. For those skilled in the art, changes, alternatives, and modifications can be made to the embodiments without departing from the scope of the present disclosure.

## Claims

1. A DNA polymerase mutant, having at least three amino acid mutations at the following four sites or functionally equivalent sites, compared with a *Pyrococcus* sp. GB-D DNA polymerase exo-mutant: site 409, site 410, site 411, and site 486,
wherein the *Pyrococcus* sp. GB-D DNA polymerase exo-mutant has the amino acid sequence as set forth in SEQ ID NO: 1.

2. The DNA polymerase mutant according to claim 1, wherein the amino acid sequence of the DNA polymerase mutant, except for site 409, site 410, site 411 and site 486, has at least 95% identity, at least 98% identity, or at least 99% identity, preferably at least 100% identity, to the amino acid sequence of the *Pyrococcus* sp. GB-D DNA polymerase exo-mutant, excluding that the DNA polymerase mutant comprise the following mutations simultaneously: L at site 409 mutated to A, Y at site 410 mutated to A, P at site 411 mutated to I, and A at site 486 mutated to L.

3. The DNA polymerase mutant according to claim 1, having the following mutations:
(1) amino acid L at site 409 mutated to A, V, Y, I, F, Q, or M;
(2) amino acid Y at site 410 mutated to A or G;
(3) amino acid P at site 411 not mutated or mutated to Q, M, N, or C; and
(4) amino acid A at site 486 mutated to N, P, T, E, L, F, K, H, R, Y, M, W, or I.

4. The DNA polymerase mutant according to claim 3, having the following mutations:
(1) amino acid L at site 409 mutated to A, V, Y, I, F, Q, or M;
(2) amino acid Y at site 410 mutated to A or G;
(3) amino acid P at site 411 not mutated or mutated to Q, M, N, or C; and
(4) amino acid A at site 486 mutated to L, F, K, H, R, Y, M, W, or I.

5. The DNA polymerase mutant according to claim 4, having any one of mutation combinations (1) to (8):
(1) amino acid Y at site 410 mutated to G, amino acid L at site 409 mutated to V, amino acid P at site 411 not mutated, and amino acid A at site 486 mutated to L; or
(2) amino acid Y at site 410 mutated to G, amino acid L at site 409 mutated to A, amino acid P at site 411 not mutated, and amino acid A at site 486 mutated to F; or
(3) amino acid Y at site 410 mutated to G, amino acid L at site 409 mutated to V, amino acid P at site 411 not mutated, and amino acid A at site 486 mutated to F; or
(4) amino acid Y at site 410 mutated to G, amino acid L at site 409 mutated to Y, amino acid P at site 411 mutated to M, and amino acid A at site 486 mutated to K; or
(5) amino acid Y at site 410 mutated to G, amino acid L at site 409 mutated to V, amino acid P at site 411 not mutated, and amino acid A at site 486 mutated to Y; or
(6) amino acid Y at site 410 mutated to A, amino acid L at site 409 mutated to A, amino acid P at site 411 mutated to Q, and amino acid A at site 486 mutated to K; or
(7) amino acid Y at site 410 mutated to A, amino acid L at site 409 mutated to A, amino acid P at site 411 not mutated, and amino acid A at site 486 mutated to L; or
(8) amino acid Y at site 410 mutated to A, amino acid L at site 409 mutated to A, amino acid P at site 411 not mutated, and amino acid A at site 486 mutated to W.

6. The DNA polymerase mutant according to claim 4, having any one of mutation combinations (1) to (11):
(1) L at site 409 mutated to A, Y at site 410 mutated to G, P at site 411 not mutated, and A at site 486 mutated to K; or
(2) L at site 409 mutated to I, Y at site 410 mutated to G, P at site 411 not mutated, and A at site 486 mutated to K; or
(3) L at site 409 mutated to A, Y at site 410 mutated to A, P at site 411 not mutated, and A at site 486 mutated to H; or
(4) L at site 409 mutated to A, Y at site 410 mutated to A, P at site 411 mutated to N, and A at site 486 mutated to K; or
(5) L at site 409 mutated to A, Y at site 410 mutated to A, P at site 411 mutated to M, and A at site 486 mutated to L; or
(6) L at site 409 mutated to M, Y at site 410 mutated to G, P at site 411 not mutated, and A at site 486 mutated to K; or
(7) L at site 409 mutated to A, Y at site 410 mutated to A, P at site 411 mutated to Q, and A at site 486 mutated to F; or
(8) L at site 409 mutated to A, Y at site 410 mutated to A, P at site 411 mutated to Q, and A at site 486 mutated to L; or
(9) L at site 409 mutated to A, Y at site 410 mutated to G, P at site 411 mutated to N, and A at site 486 mutated to K; or
(10) L at site 409 mutated to A, Y at site 410 mutated to A, P at site 411 mutated to C, and A at site 486 mutated to L; or
(11) L at site 409 mutated to A, Y at site 410 mutated to A, P at site 411 mutated to N, and A at site 486 mutated to L.

7. The DNA polymerase mutant according to claim 4, having any one of mutation combinations (1) to (3):
(1) L at site 409 mutated to Y, Y at site 410 mutated to G, P at site 411 mutated to Q, and A at site 486 mutated to K; or
(2) L at site 409 mutated to I, Y at site 410 mutated to G, P at site 411 not mutated, and A at site 486 mutated to F; or
(3) L at site 409 mutated to A, Y at site 410 mutated to A, P at site 411 mutated to Q, and A at site 486 mutated to R.

8. The DNA polymerase mutant according to claim 3, having any one of mutation combinations (1) to (10):
(1) L at site 409 mutated to V, Y at site 410 mutated to G, P at site 411 not mutated, and A at site 486 mutated to K; or
(2) L at site 409 mutated to A, Y at site 410 mutated to G, P at site 411 not mutated, and A at site 486 mutated to H; or
(3) L at site 409 mutated to F, Y at site 410 mutated to G, P at site 411 not mutated, and A at site 486 mutated to F; or
(4) L at site 409 mutated to V, Y at site 410 mutated to G, P at site 411 not mutated, and A at site 486 mutated to H; or
(5) L at site 409 mutated to Q, Y at site 410 mutated to G, P at site 411 mutated to M, and A at site 486 mutated to K; or
(6) L at site 409 mutated to Y, Y at site 410 mutated to G, P at site 411 not mutated, and A at site 486 mutated to Y; or
(7) L at site 409 mutated to A, Y at site 410 mutated to G, P at site 411 not mutated, and A at site 486 mutated to M; or
(8) L at site 409 mutated to A, Y at site 410 mutated to G, P at site 411 not mutated, and A at site 486 mutated to I; or
(9) L at site 409 mutated to A, Y at site 410 mutated to G, P at site 411 mutated to M, and A at site 486 mutated to K; or
(10) L at site 409 mutated to A, Y at site 410 mutated to G, P at site 411 mutated to Q, and A at site 486 mutated to K.

9. A nucleic acid molecule, encoding the DNA polymerase mutant according to any one of claims 1 to 8.

10. An expression vector, comprising the nucleic acid molecule according to claim 9.

11. The expression vector according to claim 10, being a non-pathogenic viral vector,
wherein the non-pathogenic viral vector comprises an adenoviral vector or a retroviral vector.

12. A recombinant cell, carrying the nucleic acid molecule according to claim 10 or the expression vector according to claim 11.

13. The recombinant cell according to claim 12, being selected from *Escherichia coli,* yeast, or mammalian cells.

14. A recombinant strain, expressing the DNA polymerase mutant according to any one of claims 1 to 8.

15. A method for obtaining a DNA polymerase mutant, the method comprising:
culturing the recombinant cell according to any one of claims 12 and 13 or the recombinant strain according to claim 14 under a condition suitable for protein expression to obtain the DNA polymerase mutant.

16. A complex, comprising:
the DNA polymerase mutant according to any one of claims 1 to 8; and
a small-molecule compound or a macromolecule,
wherein the mutant is conjugated with the small-molecule compound or the macromolecule via a chemical bond.

17. The complex according to claim 16, wherein the small-molecule compound or the macromolecule comprises a fluorescent label, fluorescein, or an antibody.

18. A method for nucleic acid synthesis, the method comprising:
subjecting a mixture of a nucleic acid template, an amplification primer, dNTPs, and the DNA polymerase mutant according to any one of claims 1 to 8 to amplification under a condition suitable for nucleic acid amplification, to obtain the nucleic acid.

19. A method for nucleic acid sequencing, the method comprising:
subjecting a mixture of a nucleic acid to be sequenced, the DNA polymerase mutant according to any one of claims 1 to 8, and modified dNTPs to amplification under a condition suitable for nucleic acid amplification, and performing fluorescence signal detection; and
determining the nucleic acid sequence of the nucleic acid to be sequenced based on a detected fluorescence signal.

20. A nucleic acid sequencing kit, comprising:
the DNA polymerase mutant according to any one of claims 1 to 8; or
the complex according to claim 16 or claim 17.

21. Use of the nucleic acid sequencing kit according to claim 20 in sequencing.

22. Use of the DNA polymerase mutant according to any one of claims 1 to 8, the nucleic acid molecule according to claim 9, the expression vector according to any one of claims 10 and 11, the recombinant cell according to any one of claims 12 and 13, the recombinant strain according to claim 14, or the complex according to any one of claims 16 and 17 in the manufacture of a product for catalyzing DNA amplification or nucleic acid sequencing.
